# EUROPEAN PATENT SPECIFICATION

(11) **EP 1 179 597 B1**
(45) Date of publication and mention of the grant of the patent: **06.06.2007**
(21) Application number: 01118456.1
(22) Date of filing: 31.07.2001
(51) Int. Cl.: C12P 13/06, C12P 13/08, C12N 9/02, C12R 1/19

(54) **Method for producing threonine and isoleucine**
Verfahren zur Herstellung von Threonin und Isoleucin
Procédé de préparation de L-thréonine ou L-isoleucine

(30) Priority: 11.08.2000 JP 2000244921
(43) Date of publication of application: 13.02.2002
(73) Proprietor: Ajinomoto Co., Inc., Chuo-ku, Tokyo (JP)
(72) Inventor: Miyata, Yuri, Kawasaki-shi, Kanagawa (JP); Nakai, Yuta, Kawasaki-shi, Kanagawa (JP); Nakanishi, Kazuo, Kawasaki-shi, Kanagawa (JP); Ito, Hisao, Kawasaki-shi, Kanagawa (JP); Kojima, Hiroyuki, Kawasaki-shi, Kanagawa (JP); Kurahashi, Osamu, Kawasaki-shi, Kanagawa (JP)
(74) Representative: Strehl Schübel-Hopf & Partner

(56) References cited:
- EP-A- 0 358 940
- EP-A- 0 593 792
- EP-A- 0 685 555
- EP-A- 0 732 011
- EP-A- 0 733 712
- EP-A- 0 872 547
- FUJITA N ET AL: "THE PRIMARY STRUCTURE OF PHOSPHOENOLPYRUVATE CARBOXYLASE OF ESCHERICHIA COLI. NUCLEOTIDE SEQUENCE OF THE PPC GENE AND DEDUCED AMINO ACID SEQUENCE" JOURNAL OF BIOCHEMISTRY, JAPANESE BIOCHEMICAL SOCIETY, TOKYO, JP, vol. 95, no. 4, 1984, pages 909-916, XP000999976 ISSN: 0021-924X
- ANDERLUND M ET AL: "EXPRESSION OF THE ESCHERICHIA POLI PNTA AND PNTB GENES, ENCODING NICOTINAMIDE NUCLEOTIDE TRANSHYDROGENASE, IN SACCHAROMYCES CEREVISIAE AND ITS EFFECT ON PRODUCT FORMATION DURING ANAEROBIC GLUCOSE FERMENTATION" APPLIED AND ENVIRONMENTAL MICROBIOLOGY, WASHINGTON,DC, US, vol. 65, no. 6, June 1999 (1999-06), pages 2333-2340, XP000856677 ISSN: 0099-2240
- PARK S M ET AL: "METABOLIC AND PHYSIOLOGICAL STUDIES OF CORYNEBACTERIUM GLUTAMICUM MUTANTS" BIOTECHNOLOGY AND BIOENGINEERING, INTERSCIENCE PUBLISHERS, LONDON, GB, vol. 55, no. 6, 1997, pages 864-879, XP000999162 ISSN: 0006-3592

## Description

### Background of the Invention

### Field of the Invention

The present invention relates to a technique used in fermentation industry, and it relates to a bacterium belonging to the genus *Escherichia* that produces L-threonine or L-isoleucine and a method for producing L-threonine or L-isoleucine using the bacterium.

### Description of the Related Art

Industrial production of L-amino acids such as L-threonine and L-isoleucine has conventionally been attained by fermentation method using microorganisms such as coryneform bacteria and bacteria belonging to the genus *Escherichia* having ability to produce such L-amino acids. As these amino acid producing bacteria, there are used strains isolated from nature, artificial mutant strains thereof or recombinant strains thereof in which L-amino acid biosynthesis enzymes are enhanced by genetic recombination in order to obtain improved productivity.

Specifically, as methods for producing L-threonine, there have been disclosed a method utilizing a mutant strain of-bacterium belonging to the genus *Escherichia* in Japanese Patent Laid-open Publication (Kokai) No. 5-304969, methods utilizing recombinant *Escherichia coli* strains in Japanese Patent Publication Nos. 1-29559, 2-109985, 56-15696 and International Patent Publication in Japanese (Kohyo) No. 3-501682, and a method utilizing a mutant strain of *Corynebacterium* bacterium in Japanese Patent Laid-open Publication No. 62-239996, and a method utilizing a mutant strain of *Corynebacterium* bacterium is reported in Japanese Patent Laid-open Publication No. 61-195695. Further, methods for producing L-threonine by utilizing strains transformed with recombinant plasmids containing the threonine operon have been disclosed in Japanese Patent Laid-open Publication Nos. 55-131397, 59-31691, 56-15696 and International Patent Publication in Japanese No. 3-501682.

Further, as methods for producing L-isoleucine, there have been disclosed a method utilizing *Escherichia coli* in Japanese Patent Laid-open Publication No. 5-130882, a method utilizing a recombinant strain of *Escherichia coli* in Japanese Patent Laid-open Publication No. 2-458, a method utilizing mutant strain of *Corynebacterium* bacterium in Japanese Patent Publication No. 3-62395, and a method utilizing a recombinant strain of *Corynebacterium* bacterium in Japanese Patent Publication (Kokoku) No. 5-47196. It is also known that L-isoleucine producing ability can be imparted by introducing *thrABC* operon containing *thrA* gene coding for aspartokinase I-homoserine dehydrogenase I derived from *Escherichia coli,* of which inhibition by L-threonine is substantially desensitized, and *ilvGMEDA* operon containing *ilvA* gene coding for threonine deaminase, of which inhibition by L-isoleucine is substantially desensitized, and from which a region required for attenuation is removed (see Japanese Patent Laid-open Publication No. 8-47397)

Meanwhile, the sequence of phosphoenolpyruvate carboxylase gene of *Escherichia coli* is known (Fujita, N., Miwa, T., Ishijima, S., Izui, K. and Katsuki H. *J. Biochem. 95*, 909-916 (1984)), and there have been disclosed phosphoenolpyruvate carboxylase of which feedback inhibition by aspartic acid is substantially desensitized and a method for utilizing a gene therefor (WO95/06114). Further, there is also known an example of enhancement of phosphoenolpyruvate carboxylase gene with the purpose of enhancement of L-glutamic acid producing ability of coryneform bacteria (Japanese Patent Laid-open Publication No. 60-87788). Furthermore, there have also been disclosed techniques of improving amino acid producing ability by enhancing a phosphoenolpyruvate carboxylase gene together with other enzyme genes. For example, an example has been reported, in which L-glutamic acid producing ability was enhanced by enhancing glutamate dehydrogenase gene, citrate synthetase gene and phosphoenolpyruvate carboxylase gene in coryneform bacteria in which α-ketoglutarate dehydrogenase gene was deleted (WO96/06180). As for *Escherichia coli,* it has been disclosed that L-threonine producing ability was not significantly increased even if a wild-type phosphoenolpyruvate carboxylase gene was introduced into an L-threonine producing strain of *Escherichia coli,* B-3996, which was transformed with a recombinant plasmid containing the threonine operon (WO95/06114).

Further, it has also been disclosed that ability to produce substances such as amino acids can be improved by increasing enzymatic activity of nicotinamide nucleotide transhydrogenase (also referred to as "transhydrogenase" hereafter) in microbial cells, and increasing reduced type nicotinamide adenine dinucleotide phosphate producing ability (W095/11985). In this reference, it is also mentioned an example of improvement of L-threonine producing ability by enhancing a transhydrogenase gene in *Escherichia coli* transformed with a recombinant plasmid containing the threonine operon. As an amino acid of which productivity is improved by elevation of transhydrogenase activity, L-isoleucine was mentioned.

### Summary of the Invention

An object of the present invention is to improve ability to produce L-threonine or L-isoleucine of bacteria belonging to the genus *Escherichia.*

The inventors of the present invention found that the ability to produce L-threonine or L-isoleucine was markedly increased by enhancing both of phosphoenolpyruvate carboxylase activity and transhydrogenase activity, and further found that the producing ability was further improved by enhancing aspartase activity. Thus, they accomplished the present invention.

That is, the present invention provides the followings.
(1) A bacterium belonging to the genus *Escherichia*, which has an ability to produce L-threonine or L-isoleucine, and' in which intracellular phosphoenolpyruvate carboxylase activity and transhydrogenase activity are enhanced.
(2) The bacterium belonging to the genus *Escherichia* according to (1), in which activity of an enzyme or enzymes encoded by threonine operon or a part thereof is enhanced, and which has L-threonine producing ability.
(3) The bacterium belonging to the genus *Escherichia* according to (2), wherein the threonine operon consists of *thrABC*.
(4) The bacterium belonging to the genus *Escherichia* according to (1), in which activity of an enzyme or enzymes encoded by *ilv* operon or a part thereof is enhanced, and which has L-isoleucine producing ability.
(5) The bacterium belonging to the genus *Escherichia* according to any one of (1) to (4), wherein aspartase activity is enhanced.
(6) The bacterium belonging to the genus *Escherichia* according to any one of (1) to (5), wherein activity of each enzyme is enhanced by increasing copy number of a gene or operon coding for each enzyme, or modifying an expression regulatory sequence so that intracellular expression of the gene or operon should be enhanced.
(7). The bacterium belonging to the genus *Escherichia* according to (6), wherein the gene is derived from a bacterium belonging to the genus *Escherichia*.
(8) A method for producing L-threonine or L-isoleucine, which comprises culturing a bacterium belonging to the genus *Escherichia* according to any one of (1) to (7) in a medium to produce and accumulate L-threonine or L-isoleucine in the medium, and collecting the L-threonine or L-isoleucine from the medium.

According to the present invention, L-threonine or L-isoleucine producing ability of bacteria belonging to the genus *Escherichia* can be improved.

### Brief Explanation of the Drawings

Fig. 1 shows the construction of the plasmid pMW118::aspA containing *aspA* gene.
Fig. 2 shows the construction of the plasmid containing *pntAB* gene and *ppc* gene (pPTS).
Fig. 3 shows the construction of the plasmid containing *aspA* gene and *ppc* gene (pAPW).
Fig. 4 shows the construction of the plasmid containing *aspA* gene, *pntAB* gene and *ppc* gene (pAPT).
Fig. 5 shows the construction of the plasmid pHSGSK.
Fig. 6 shows the construction of the plasmid pdGM1.
Fig. 7 shows the construction of the plasmid pMWGMA2.
Fig. 8 shows the construction of the plasmid pMWD5.
Fig. 9 shows the construction of pMWD5-aspA, pMWD5-THY, pMWD5-ppc, pMWD5-PTS and pMWD5-APT.

### Detailed Description of the Invention

Hereafter, the present invention will be explained in detail.

A bacterium belonging to the genus *Escherichia* of the present invention is a bacterium belonging to the genus *Escherichia* which has an ability to produce L-threonine or L-isoleucine, and has enhanced intracellular phosphoenolpyruvate carboxylase (also abbreviated as "PEPC" hereafter) activity and transhydrogenase (also abbreviated as "THY" hereafter) activity.

As the bacteria belonging to the genus *Escherichia,* specifically, those mentioned in the work of Neidhardt *et al.* (Neidhardt, F.C. *et al., Escherichia coli* and *Salmonella Typhimurium,* American Society for Microbiology, Washington D.C., 1208, Table 1) can be used. For example, *Escherichia coli* can be mentioned.

The expression "having ability to produce L-threonine or L-isoleucine" used herein means that, when the bacterium of interest is cultured in a medium, it shows an ability to accumulate L-threonine or L-isoleucine in the medium. This L-threonine or L-isoleucine producing ability may be a property possessed by a wild strain or a property imparted or enhanced by breeding.

In the bacterium belonging to the genus *Escherichia* of the present invention, intracellular aspartase (L-aspartate ammonia-lyase, also referred to as "AspA" hereinafter) activity may be further enhanced.

In order to enhance activity of PEPC, THY or AspA in bacteria belonging to the genus *Escherichia,* a gene coding for PEPC, THY or AspA can be cloned on a suitable plasmid, and a bacterium belonging to the genus *Escherichia* that serves as a host can be transformed with the obtained plasmid. This increases copy number of a gene coding for PEPC, THY or AspA (hereafter abbreviated as *"ppc* gene", *"pntAB* gene" and "*apsA* gene", respectively, in that order) in the transformant, and as a result, the activity of PEPC, THY or AspA is enhanced.

The *ppc* gene, *pntAB* gene and *apsA* gene are introduced into a bacterium belonging to the genus *Escherichia* as a combination of the *ppc* gene and *pntAB* gene, or a combination of these genes and the *aspA* gene. These genes may be introduced into a host as one kind of plasmid in which two or three of the genes are cloned, or two or three kinds of plasmids that can coexist, in which the genes are respectively cloned.

The enhancement of PEPC, THY or AspA activity can also be attained by allowing existence of multiple copies of the *ppc* gene, *pntAB* gene or *apsA* gene on chromosomal DNA of the original parent strain that serves as a host. In order to introduce multiple copies of the *ppc* gene, *pntAB* gene or *apsA* gene into chromosomal DNA of a bacterium belonging to the genus *Escherichia,* a sequence of which multiple copies exist in the chromosomal DNA, for example, repetitive DNA, inverted repeats existing at the end of a transposable element *etc.,* can be used. Alternatively, it is also possible to incorporate the *ppc* gene, *pntAB* gene or *apsA* gene into transposon, and allow its transfer to introduce multiple copies of each gene into the chromosomal DNA. By either method, the number of copies of the *ppc* gene, *pntAB* gene or *apsA* gene within cells of the transformant strain increases, and as a result, PEPC, THY or AspA activity is enhanced.

The enhancement of PEPC, THY or AspA activity can also be attained by, besides being based on the aforementioned gene amplification, replacing an expression regulatory sequence of *ppc* gene, *pntAB* gene or *apsA* gene such as a promoter with a stronger one (see Japanese Patent Laid-open Publication No. 1-215280). For example, *lac* promoter, *trp* promoter, trc promoter, *tac* promoter, *P_{R}* promoter and *P_{L}* promoter of lambda phage, tet promoter, *amyE* promoter, *spac* promoter and so forth are known as strong promoters. Substitution of these promoters enhances expression of the *ppc* gene, *pntAB* gene or *apsA* gene, and hence the PEPC, THY or AspA activity is enhanced. Enhancement of an expression regulatory sequence may be combined with increasing copy number of the *ppc* gene, *pntAB* gene or *apsA* gene.

The organism as the source of the *ppc* gene, *pntAB* gene or *apsA* gene may be any organism having the PEPC, THY or AspA activity. Particularly preferred are bacteria that are prokaryotes, for example, bacteria belonging to the genus *Enterobacter, Klebsiella, Erwinia, Serratia, Escherichia, Corynebacterium, Brevibacterium* or *Bacillus.* As a specific example, *Escherichia coli* can be mentioned. The *ppc* gene, *pntAB* gene or *apsA* gene can be obtained from chromosomal DNA of such microorganisms as mentioned above.

The *ppc* gene of *Escherichia coli* can be obtained from a plasmid having this gene, plasmid pS2 (Sabe, H. et al., *Gene, 31,* 279 (1984)) or pT2. By digesting pS2 with *Aat*II and *Afl*II, a DNA fragment containing the *ppc* gene can be obtained. A DNA fragment having the *ppc* gene can also be obtained by digesting pT2 with *Sma*I and *Sca*I*.* The *E. coli* F15 strain (AJ12873) harboring pT2 was deposited on July 15, 1993 at the National Institute of Bioscience and Human-Technology, Agency of Industrial Science and Technology (1-3, Higashi 1-chome, Tsukuba-shi, Ibaraki-ken, Japan, postal code: 305-8566) (currently, the independent administrative corporation, the National Institute of Advanced Industrial Science and Technology, International Patent Organism Depositary (Chuo Dai-6, 1-1 Higashi 1-Chome, Tsukuba-shi, Ibaraki-ken, Japan, postal code: 305-5466) and received an accession number of FERM P-13752. Then, it was transferred to an international deposit under the provisions of the Budapest treaty on July 11, 1994, and received an accession number of FERM BP-4732.

The *pntAB* gene can be obtained by digesting the plasmid pMW::THY (W095/11985) containing the gene with *Sma*I and *Hind*III. The *Escherichia coli* AJ12929 strain harboring pMW::THY was deposited at the National Institute of Bioscience and Human-Technology, Agency of Industrial Science and Technology, Ministry of International Trade and Industry (postal code 305-8566, 1-3 Higashi 1-chome, Tsukuba-shi, Ibaraki-ken, Japan) on October 47 1993, and received an accession number of FERM P-13890. Then, it was transferred from the above original deposit to an international deposit under the provisions of the Budapest Treaty on September 14, 1994, and received an accession number of FERM BP-4798. The transhydrogenase of *Escherichia coli* consists of two subunits, which are encoded by *pntA* and *pntB*, respectively.

While the bacterium belonging to the genus *Escherichia* of the present invention is not particularly limited so long as it has the L-threonine or L-isoleucine producing ability, specific examples thereof include, for example, bacteria belonging to the genus *Escherichia* imparted with the L-threonine producing ability by enhancing activity of an enzyme encoded by the threonine operon or a part thereof and in addition, bacteria belonging to the genus *Escherichia* imparted with the L-isoleucine producing ability by enhancing activity of an enzyme encoded by the *ilv* operon or a part thereof.

The threonine operon or a part thereof may be, for example, *thrABC* or a part thereof. The *ilv* operon or a part thereof may be, for example, *ilvGMEDA* or a part thereof.

As *Escherichia coli* having L-threonine producing ability, there can be specifically mentioned *Escherichia coli* VKPM B-3996 (deposited on November 19, 1987 at All-Union Scientific Center of Antibiotics, Nagatinskaya Street 3-A, 113105, Moscow, Russian Federation with a registration number of RIA 1867, see U.S. Patent No. 5,175,107), *Escherichia coli* AJ11335 (Japanese Patent Laid-open Publication No. 55-131397) and so forth. The VKPM B-3996 strain harbors a plasmid pVIC40 (International Patent Publication WO90/04636), which is obtained by inserting a threonine biosynthesis system gene (threonine operon: *thrABC*) into a wide host-range vector plasmid having a streptomycin resistance marker, pAYC32 (see Chistorerdov, A.Y., Tsygankov, Y.D., *Plasmid, 1986,* 16, 161-167). The feedback inhibition by L-threonine of the aspartokinase I-homoserine dehydrogenase I encoded by *thrA* in that operon is desensitized.

As bacteria belonging to the genus *Escherichia* having L-isoleucine producing ability, the *Escherichia coli* KX141 (VKPM B-4781, see European Patent Laid-open Publication No. 519,113) and *Escherichia coli* AJ12919 (Japanese Patent Laid-open Publication No. 8-47397) can be mentioned. The VKPM B-3996 strain in which the *ilv* operon is amplified is also a preferred L-isoleucine producing bacterium.

The threonine operon contains the *thrA, thrB* and *thrC* genes, and they code for aspartokinase I-homoserine dehydrogenase I, homoserine kinase and threonine synthase, respectively, in that order. As for these enzymes, it is preferred that the inhibition of aspartokinase I-homoserine dehydrogenase I by L-threonine should be substantially desensitized.

The *ilvGMEDA* operon contains the *ilvG, ilvM, ilvE, ilvD* and *ilvA* genes, and they code for the large subunit, small subunit, transaminase, dihydroxy-acid dehydratase and threonine deaminase of isozyme II of acetohydroxy-acid synthase, respectively, in that order. Since the *ilvGMEDA* operon is under control (attenuation) of expression of the operon by L-valine and/or L-isoleucine and/or L-leucine, a region required for the attenuation may be removed or mutated in an L-isoleucine producing bacterium in order to desensitize suppression of the expression by the produced L-isoleucine. As the *ilvGMEDA* operon, those derived from bacteria belonging to the genus *Escherichia,* in particular, the *ilvGMEDA* operon derived from *E. coli,* can be mentioned. The *ilvGMEDA* operon is detailed in WO96/26289. As for the *ilvGMEDA* operon, it is preferred that the region required for attenuation should be removed,'and among the enzymes encoded by this operon, inhibition of threonine deaminase by L-isoleucine should be substantially desensitized (see Japanese Patent Laid-open Publication No. 8-47397).

Enhancement of activities of the enzymes encoded by the threonine operon or *ilv* operons or a part thereof may be attained in the same manner as that for PEPC, THY and AspA.

In a microorganism used for the present invention, if a gene for an enzyme responsible for a pathway involved in biosynthesis of target amino acid is enhanced, or a gene or operon coding for a desensitized type (inhibition desensitized type) enzyme of an enzyme suffering from feedback inhibition is introduced, the L-amino acid producing ability may further be improved.

Threonine or isoleucine can be produced by culturing a bacterium belonging to the genus *Escherichia* in which PEPC and THY as well as AspA, if required, are enhanced as described above and which has an ability to produce L-threonine or L-isoleucine in a medium to produce and accumulate threonine or isoleucine in the medium, and collecting the threonine or isoleucine from the medium.

The medium used for the culture may be a usual medium containing a carbon source, nitrogen source, inorganic ions, and other organic components as required.

As the carbon source, it is possible to use sugars such as glucose, lactose, galactose, fructose and starch hydrolysate; alcohols such as glycerol and sorbitol; or organic acids such as fumaric acid, citric acid and succinic acid.

As the nitrogen source, it is possible to use inorganic ammonium salts such as ammonium sulfate, ammonium chloride or ammonium phosphate; organic nitrogen such as soybean hydrolysate; ammonia gas; or aqueous ammonia.

As for the organic trace nutrients, it is desirable to add required substances such as vitamin B₁, yeast extract and so forth in a suitable amount. In addition to these, small amounts of potassium phosphate, magnesium sulfate, iron ions, manganese ions and so forth are added.

Culture is preferably carried out under an aerobic condition for 16-72 hours. The culture temperature is controlled to be 25°C to 45°C, and pH is controlled to be 5 to 8 during the culture. Inorganic or organic, acidic or alkaline substances as well as ammonia gas and so forth can be used for pH adjustment.

Collection of L-threonine or L-isoleucine from fermented liquor is usually carried out by a combination of an ion exchange resin technique, precipitation and other known techniques.

### Best Mode for Carrying out the Invention

The present invention will be further specifically explained hereinafter with reference to the following examples.

### Example 1: Production of plasmids containing various genes

### (1) Production of plasmid containing aspA gene (pMW118::aspA)

A DNA fragment containing the *aspA* gene was amplified by PCR using chromosomal DNA of the *Escherichia coli* W3110 strain as a template and the following primers.
Primer 1: 5'-TGATCAGCGAAACACTTTTA-3' (SEQ ID NO: 1)
Primer 2: 5'-CAGCAAACTATGATGAGAA-3' (SEQ ID NO: 2)

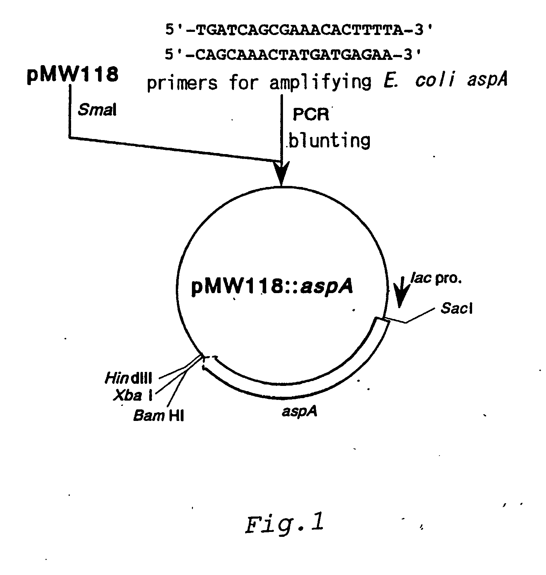

The obtained amplified fragment was inserted into the SmaI cleavage site of pMW118 (Nippon Gene) to obtain pMW118::aspA (Fig. 1).

### (2) Production of plasmid containing pntAB gene and ppc gene (pPTS)

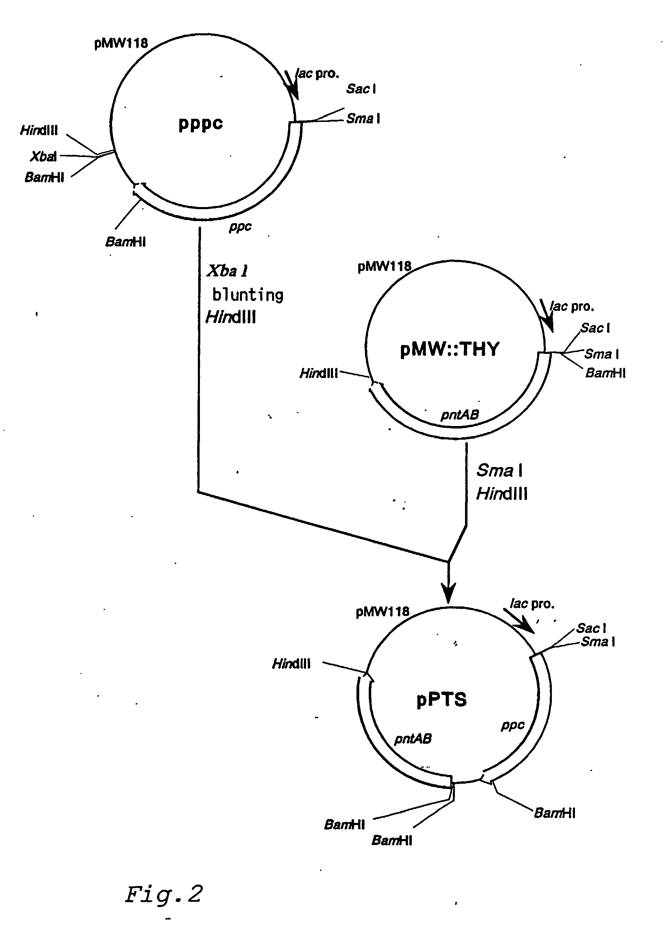

The plasmid pMW::THY containing the *pntAB* gene described in W095/11985 was digested with *Sma*I and *Hind*III*,* and a DNA fragment containing *pntAB* was collected. Then, the plasmid pppc containing the *ppc* gene described in W095/16042 was digested with *Xba*I. After the both ends were blunt-ended, it was further digested with *Hind*III, and inserted with the above DNA fragment containing *pntAB* at the cleavage site to obtain a plasmid pPTS (Fig. 2).

### (3) Production of plasmid containing aspA gene and ppc gene (pAPW)

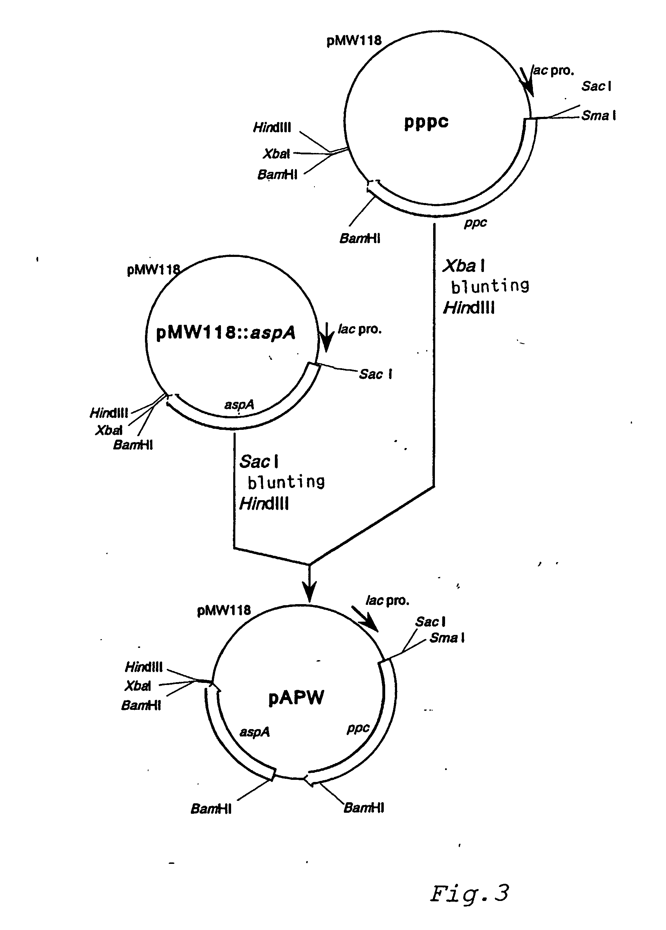

pMW118::aspA was digested with *Sac*I, and the both ends were_blunt-ended. It was further digested with *Hind*III to obtain a DNA fragment containing *aspA*. Then, the aforementioned pppc was digested with *Xba*I, and the both ends were blunt-ended. It was further digested with *Hind*III, and inserted with the aforementioned DNA fragment containing *aspA* at the cleavage site to obtain pAPW (Fig. 3).

### (4) Production of plasmid containing aspA gene, pntAB gene, and ppc gene (pAPT)

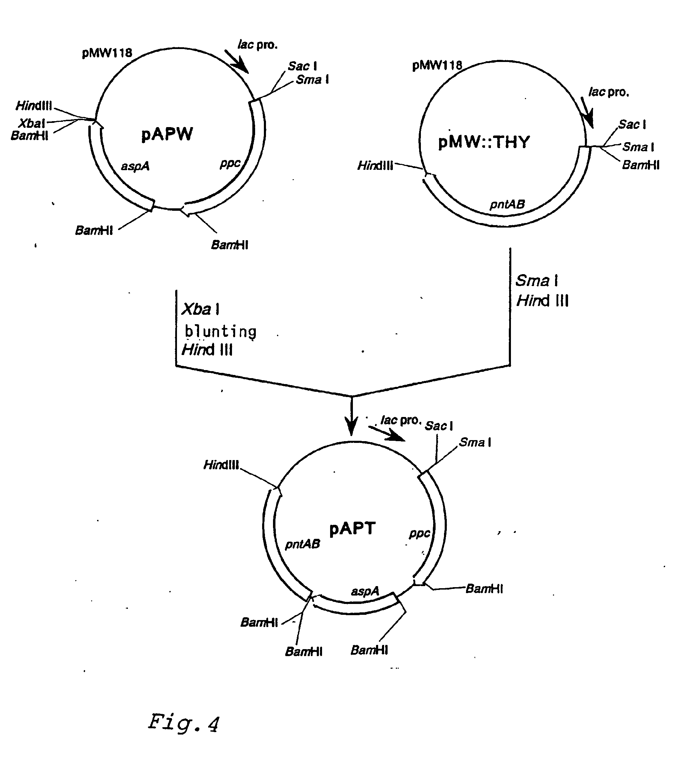

A DNA fragment containing *pntAB* was obtained by digesting pMW::THY with *Sma*I and *Hind*III. Then, the aforementioned pAPW was digested with *Xba*I, and the both ends were blunt-ended. It was further digested with *Hind*III and inserted with the aforementioned *pntAB* at the cleavage site to obtain pAPT (Fig. 4).

### (5) Production of plasmid containing ilvGMEDA operon (pMWD5)

A DNA fragment containing *ilvGMEDA* operon was prepared from the plasmid pMWD5 containing the ilvGMED operon, which is disclosed in WO96/26289. The plasmid pMWD5 was constructed as follows.

The chromosomal DNA was extracted from *Escherichia coli* MI162. The chromosomal DNA was cleaved with restriction enzyme *Hin*dIII. The length of a *Hin*dIII-*Hin*dIII DNA fragment including *ilvGM* genes was found to be 4.8 kb. Therefore, the *Hin*dIII-*Hin*dIII DNA fragment with approximately 4.8 kb and the DNA fragment obtained by digestion of the plasmid vector pBR322 (purchased form Takara Shuzo, Co., Ltd.) with *Hin*dIII, were ligated.

The resulting DNA-ligated mixture was induced into *Escherichia coli* MI162 which is an acetohydroxy-acid synthase-deficient strain. The strains in which the deficiency of acetohydroxy-acid synthase was complemented by transformation were selected and the plasmid structure was isolated from the selected strains. The results of the analysis of the plasmid revealed that a 4.8-kb DNA fragment containing the *ilvGM* gene and a portion of 5'-terminal of *ilvE* gene was inserted into the *Hind*III site of the pBR322. The plasmid was termed pBRGM7.

The synthetic oligonucleotides shown in SEQ ID NO:3 and NO:4 were synthesized with reference to the DNA sequence of the *ilvGM* gene described in Gene, *97,* 21, (1991), *Pro. Natl. Acad. Sci. U.S.A., 78,* 922, (1981) and *J. Bacteriol., 149,* 294, (1982). DNA was amplified by the PCR method, using both oligonucleotides as primers and chromosomal DNA of MI162 strain as a template. The amplified fragment was termed Fragment (A).

Similarly, the synthetic oligonucleotides shown in SEQ ID NO:5 and NO:6 were synthesized with reference to the DNA sequence described in Gene, *97,* 21, (1991), *Pro. Natl. Acad. Sci. U.S.A., 78,* 922, (1981) and *J. Bacteriol.*, *149,* 294, (1982). DNA was amplified by the PCR method, using both synthesized DNAs as primers and chromosomal DNA of the MI162 strain as a template. The amplified DNA fragment was termed Fragment (B).

The plasmid pUCA was prepared by ligating the large fragment obtained by digestion of Fragment (A) with *Sma*I and the DNA fragment obtained by digestion of the vector, pUC18 (Takara Shuzo, Co., Ltd.) with *Sma*I. The plasmid pHSGB was prepared by ligating the large fragment obtained by digestion of Fragment (B) with *Kpn*I and the DNA fragment obtained by digestion of the vector, pHSG399 (Takara Shuzo, Co., Ltd.) with *Hinc*II and *Kpn*I*.*

The plasmid pUCA was digested with *Kpn*I*,* the blunt-end fragment was prepared with the large fragment of DNA polymerase I (Klenow fragment), and digested with *PstI,* and finally, a DNA fragment containing Fragment (A) was isolated. Plasmid pHSGB was digested with HindIII, the blunt-end fragment was prepared with the large fragment of DNA polymerase I (Klenow fragment), and digested with *Pst*I, and finally, a DNA fragment containing Fragment (B) was isolated. The plasmid pHSGSK was prepared by ligating both DNA fragments.

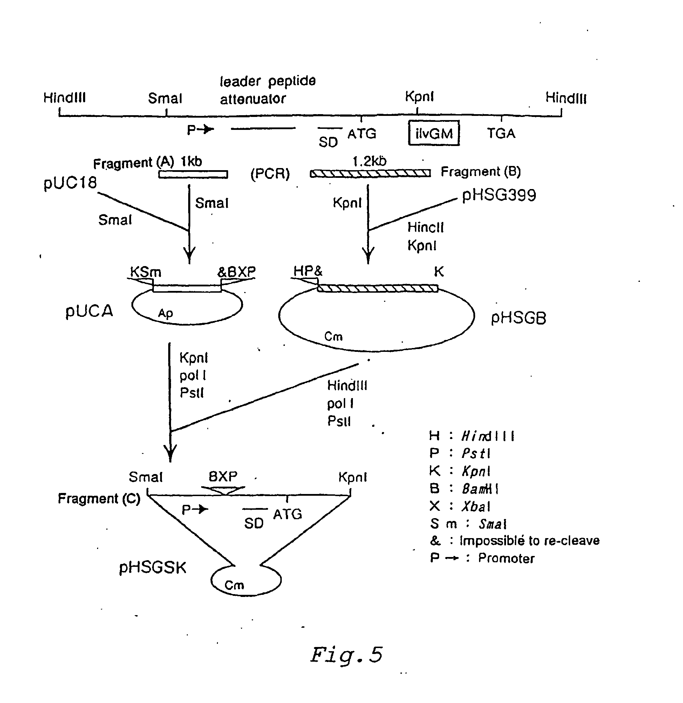

The *Sma*I-*Kpn*I fragment derived from Fragments (A) and (B) in pHSGSK was termed Fragment (C). Fragment (C) corresponded to a fragment obtained by digestion of a 4.8-kb *Hind*III-*Hind*III fragment with *Sma*I and *Kpn*I, contained a promoter, the SD sequence and a upstream region of the *ilvG* gene, but lost the DNA sequence of 0.2 kb from a leader sequence to an attenuator. The scheme of construction of pHSGSK is summarized in Fig. 5.

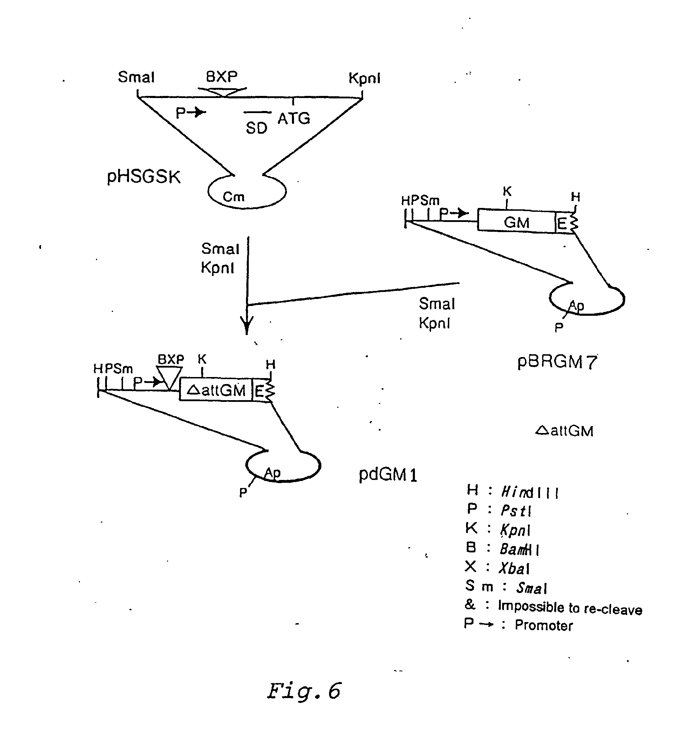

Fragment (C) was obtained by digestion of the plasmid pHSGSK with *Sma*I and *Kpn*I, the large DNA fragment was obtained by digestion of the plasmid pBRGM7 with *Sma*I and *Kpn*I, and the both two fragments were ligated. The obtained plasmid was termed pdGM1. pdGM1 harbored a 4.6-kb *Hin*dIII-*Hin*dIII fragment including the *ilvGM* gene, which lost the region necessary for attenuation. This *ilvGM* gene which loses the region necessary for attenuation represents " attGM". The scheme of the construction of pdGM1 is summarized in Figure 6.

The plasmid pDRIA4 described in Japanese Patent Application Laid-Open No. 2-458(1990) is prepared by combining the shuttle vector pDR1120, which allows autonomous replication in both a microorganism belonging to the genus *Escherichia* and a microorganism belonging to the genus *Brevibacterium,* with a *Bam*HI-*Bam*HI fragment including the *ilvA* gene encoding threonine deaminase and a portion of the 3'-terminal of the *ilvD* gene derived from *E. coli* K-12. Japanese Patent Application Laid-Open No. 2-458(1990) describes that the length of the *Bam*HI-*Bam*HI fragment is 2.3 kb; however, at present, the length of this fragment has been found to be 2.75 kb. The plasmid pDRIA4 is not present within the chromosomal DNA of *Brevibacterium flavum* AJ12358 (FERM P-9764) or *Brevibacterium flavum* AJ12359 (FERM P-9765). From these strains, the plasmid pDRIA4 can be prepared according to the usual method.

From a 2.75-kb *Bam*HI*-Bam*HI DNA fragment in the plasmid pDRIA4, a HindIII-BamHI fragment including the *ilvA* gene encoding threonine deaminase, in which the inhibition by L-isoleucine was released, was prepared, and ligated to a DNA fragment obtained by cleaving the vector pMW119 (NIPPON GENE) with *Hin*dIII and *Bam*HI. The resulting plasmid was termed pMWA1.

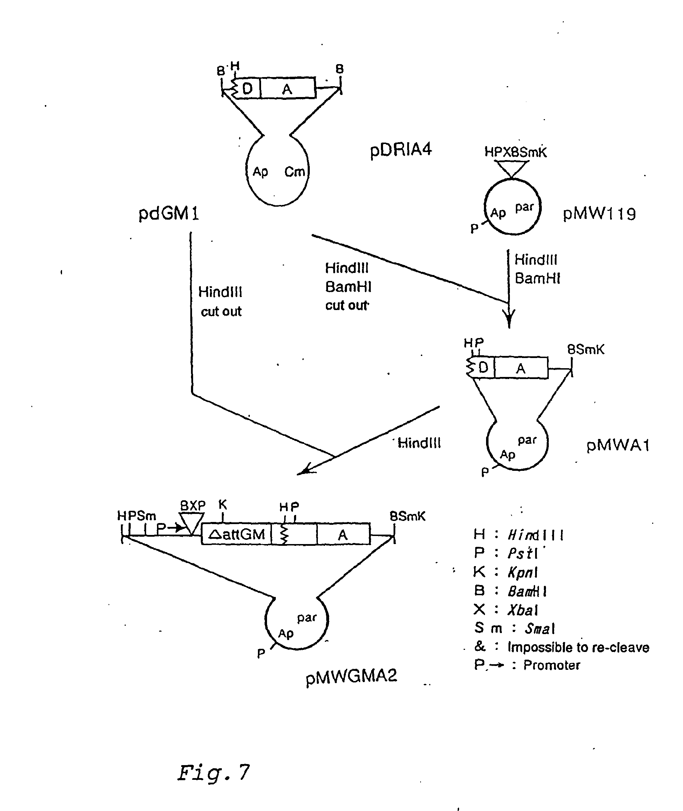

A DNA fragment obtained by cleaving the plasmid pMWA1 with *Hin*dIII and a DNA fragment obtained by cleaving the plasmid pdGM1 with *Hin*dIII were ligated. According to the analysis of the position of the restriction sites of the ligated plasmids, the plasmid in which the transcriptional orientations of the *ilvGM* and *ilvA* genes were the same was selected, and termed pMWGMA2. The pMWGMA2 includes the *ilvGM* gene in which an attenuator was deleted, a 5'-terminal portion of the *ilvE* gene, and a 3'-terminal portion of the *ilvD* gene. The scheme of the construction of pMWGMA2 is summarized in Figure 7.

The chromosomal DNA of *Escherichia coli* MI162 was prepared and cleaved with *Sal*I and *Pst*I to prepare the mixture of DNA fragments. On the other hand, a DNA fragment was prepared by cleaving the vector pUC19 (Takara Shuzo, Co., Ltd.) with *Sal*I and *Pst*I. The mixture of DNA fragments was ligated to the DNA fragment obtained by cleaving pUC19, and the DNA mixture was obtained. The DNA mixture was induced into AB2070, a transaminase B-deficient strain, (provided from *Escherichia coli* Genetics Stock Center. J. Bacteriol., *109,* 703, (1972), CGSC2070) and a transformant, in which the branched-chain amino-acid requirement was recovered, was selected. As a result of the preparation of a plasmid from the strain, the plasmid harbored a DNA fragment obtained by cleaving the plasmid pUC19 with *Sal*I and *Pst*I, and a *Sal*I-*Pst*I DNA fragment including the *ilvE* gene, which were ligated. The plasmid was termed pUCE1. The pUCE1 includes a 3'-terminal portion of the *ilvM* gene, the *ilvE* gene, and a 5'-terminal portion of the *ilvD* gene.

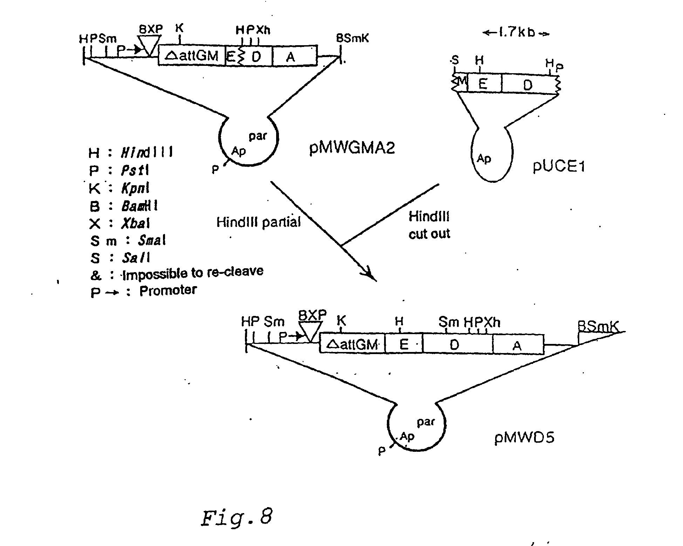

A DNA-fragment mixture was prepared by partially digesting pMWGMA2 with *Hin*dIII. On the other hand, a 1.7-kb *Hin*dIII-*Hin*dIII DNA fragment containing a portion of the *ilvE* gene and a 5'-terminal portion of the *ilvD* gene was prepared by cleaving pUCE1 with *Hin*dIII. Using a DNA mixture obtained by ligating both of the DNA fragments, AB1280, a dihydroxy-acid dehydratase(*ilvD* gene product)-deficient strain, was transformed, and the strain which recovered branched chain amino acid requirement was selected from the transformants. In the plasmid prepared from the resulting transformant, a DNA fragment obtained by cleaving only the *Hin*dIII site between attGM and *ilvA* of pMWGMA2 with *Hin*dIII*,* and a 1.7-kb *Hin*dIII-*Hin*dIII DNA fragment including a portion of the *ilvE* gene and a portion of the *ilvD* gene derived from pUCE1 were ligated, and the *ilvGMEDA* operon was reconstructed. The plasmid was termed pMWD5. The scheme of the construction of pMWD5 is summarized in Figure 8.

The resulting plasmid pMWD5 derived from the vector pMW119 harbors the *ilvGMEDA* operon in which the region necessary for attenuation is deleted.

The plasmid pMWD5 (Ap^{r}) obtained as described above is a plasmid containing pMW119 as a vector and carrying the *ilvGMEDA* operon from which the region required for attenuation was removed.

### (6) Production of plasmid containing ilvGMEDA operon and aspA gene (pMWD5-aspA)

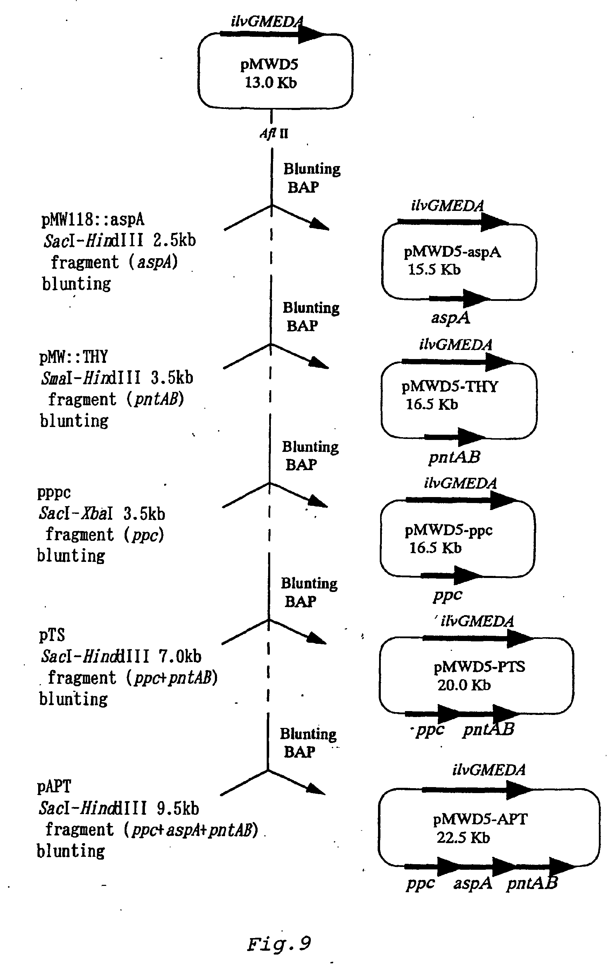

pMW118::aspA was digested with *Sac*I and *Hind*III*,* and blunt-ended to obtain a DNA fragment containing the *aspA.* pMWD5 was digested with *Afl*II, blunt-ended and inserted at the cleavage site with the above DNA fragment containing *aspA* to obtain pMWD5-aspA (Fig. 9).

### (7) Production of plasmid containing ilvGMEDA operon and pntAB gene (pMWD5-THY)

pMW::THY was digested with *Sma*I and *Hind*III, and blunt-ended to obtain a DNA fragment containing *pntAB*. pMWD5 was digested with *Afl*II, blunt-ended, and inserted at the cleavage site with the above DNA fragment containing the *pntAB* to obtain pMWD5-THY (Fig. 9).

### (8) Production of plasmid containing ilvGMEDA operon and ppc gene (pMWD5-ppc)

pppc was digested with *Sac*I and *Xba*I, and blunt-ended to obtain a DNA fragment containing *ppc.* pMWD5 was digested with *AflII,* blunt-ended and inserted at the cleavage site with the above DNA fragment containing *ppc* to obtain pMWD5-ppc (Fig. 9).

### (9) Production of plasmid containing ilvGMEDA operon, pntAB gene and ppc gene (pMWD5-PTS)

pPTS was digested with *Sac*I and *Hind*III, and blunt-ended to obtain a DNA fragment containing *ppc* and *pntAB.* pMWD5 was digested with *Afl*II, blunt-ended, and inserted at the cleavage site with the above DNA fragment containing *ppc* and *pntAB* to obtain pMWD5-PTS (Fig. 9).

### (10) Production of plasmid containing ilvGMEDA operon, aspA gene, pntAB gene and ppc gene (pMWD5-APT)

pAPT was digested with *Sac*I and *Hind*III, and blunt-ended to obtain a DNA fragment containing *ppc, pntAB* and *asp*A. pMWD5 was digested with *Afl*II, blunt-ended and inserted at the cleavage site with the above DNA fragment containing *ppc, pntAB*, and *aspA* to obtain pMWD5-APT (Fig. 9).

### Example 2: Production of amino acids by Escherichia coli harboring various plasmids

### (1) Production of L-threonine

The various plasmids obtained in Example 1 were each introduced into *Escherichia coli* VKPM B-3996. These strains were cultured under the following conditions.

The culture was performed for 38 hours at 37°C with stirring at 114-116 rpm by using a medium having the composition shown in Table 1. Component A, Component B and Component C mentioned in Table 1 were prepared and sterilized separately, and then they were cooled and mixed in a ratio of 16/20 volume of Component A, 4/20 volume of Component B and 30 g/L of Component C. The results of measurement of the accumulated amounts of L-threonine in the medium are shown in Table 2. It was found that, in L-threonine producing bacteria belonging to the genus *Escherichia,* L-threonine productivity could be improved by enhancing intracellular THY activity and PEPC activity. Further, it was also found that L-threonine productivity could be further improved by enhancing AspA activity.

**Table 1: Threonine production medium**

| | | |
|---|---|---|
| A | | (g/L) |
| | (NH₄)₂SO₄ | 16 |
| | KH₂PO₄ | 1 |
| | MgSO₄·7H₂O | 1 |
| | FeSO₄·7H₂O | 0.01 |
| | MnSO₄·4H₂O | 0.01 |
| | Yeast Extract (Difco) | 2 |
| | L-Methionine | 0.5 |
| | adjusted to pH 7.0 with KOH and autoclaved at 115°C for 10 minute (16/20 volume) | |
| B | 20% glucose autoclaved at 115°C for 10 minute (4/20 volume) | |
| C | CaCO₃ according to Japanese Pharmacopoeia, subjected to dry sterilization at 180°C for 2 days (30 g/L) antibiotics (100 µg/L of streptomycin and 50 µg/L of ampicillin) | |

**Table 2**

| Host | Plasmid | Accumulated amount of L-threonine (g/L) |
|---|---|---|
| B-3996 | pMW118 | 14.0 |
| | pppc | 14.5 |
| | pMW::THY | 15.0 |
| | PMW118::aspA | 14.0 |
| | pPTS | 16.8 |
| | pAPT | 17.2 |

### (2) Production of L-isoleucine

The various plasmids obtained in Example 1 were each introduced into *Escherichia coli* VKPM B-3996. These strains were cultured under the following conditions.

The culture was performed in a medium for L-isoleucine production (containing 40 g glucose, 16 g of ammonium sulfate, 1 g of monopotassium phosphate, 1 g of magnesium sulfate heptahydrate, 0.01 g of ferrous sulfate heptahydrate, 0.01 g of manganese chloride tetrahydrate, 2 g of yeast extract and 40 g of calcium carbonate in 1 L of water, pH = 7.0) at 37°C for 24 hours. L-Isoleucine contained in the medium was quantified by high performance liquid chromatography. The results are shown in Table 3.

It was found that, in L-threonine producing bacteria belonging to the genus *Escherichia*, L-isoleucine productivity could be improved by enhancing intracellular THY activity and PEPC activity. Further, it was also found that L-isoleucine productivity could be further improved by enhancing AspA activity.

**Table 3**

| Host | Plasmid | Accumulated amount of L-isoleucine (g/L) |
|---|---|---|
| B-3996 | pMWD5 | 10.0 |
| | pMWD5-ppc | 9.9 |
| | pMWD5-THY | 10.4 |
| | pMWD5-aspA | 10.0 |
| | pMWD5-PTS | 10.8 |
| | pMWD5-APT | 11.2 |

## Claims

1. A bacterium belonging to the genus *Escherichia,* which has an ability to produce L-threonine or L-isoleucine, and in which intracellular phosphoenolpyruvate carboxylase activity and transhydrogenase activity are enhanced by increasing copy number of a gene or operon coding for each enzyme, or modifying an expression regulatory sequence so that intracellular expression of the gene or operon should be enhanced.

2. The bacterium belonging to the genus *Escherichia* according to claim 1, in which activity of an enzyme or enzymes encoded by threonine operon or a part thereof is enhanced by increasing copy number of a gene or operon coding for each enzyme, or modifying an expression regulatory sequence so that intracellular expression of the gene or operon should be enhanced, and which has L-threonine producing ability.

3. The bacterium belonging to the genus *Escherichia* according to claim 2, wherein the threonine operon consists of *thrABC*.

4. The bacterium belonging to the genus *Escherichia* according to claim 1, in which activity of an enzyme or enzymes encoded by *ilv* operon or a part thereof is enhanced by increasing copy number of a gene or operon coding for each enzyme, or modifying an expression regulatory sequence so that intracellular expression of the gene or operon should be enhanced, and which has L-isoleucine producing ability.

5. The bacterium belonging to the genus *Escherichia* according to any one of Claims 1-4, wherein aspartase activity is enhanced by increasing copy number of a gene or operon coding for each enzyme, or modifying an expression regulatory sequence so that intracellular expression of the gene or operon should be enhanced.

6. The bacterium belonging to the genus *Escherichia* according to any of claims 1-5, wherein each of the gene or operon is derived from a bacterium belonging to the genus *Escherichia.*

7. A method for producing L-threonine or L-isoleucine, which comprises culturing a bacterium belonging to the genus *Escherichia* according to any one of claims 1-6 in a medium.

## Patentansprüche

1. Bakterium der Gattung Escherichia, das die Fähigkeit zur Produktion von L-Threonin oder L-Isoleucin aufweist und worin die intrazelluläre Phosphoenolpyruvatcarboxylaseaktivität und die Transhydrogenaseaktivität **dadurch** verstärkt sind, dass die Kopienzahl eines Gens oder Operons, das für das jeweilige Enzym kodiert, erhöht ist oder die Expressionsregulationssequenz so modifiziert ist, dass die intrazelluläre Expression des Gens oder Operons verstärkt sein sollte.

2. Bakterium der Gattung Escherichia nach Anspruch 1, worin die Aktivität eines Enzyms oder von Enzymen, kodiert von dem Threoninoperon oder einem Teil davon, **dadurch** verstärkt ist, dass die Kopienzahl eines Gens oder Operons, das für das jeweilige Enzym kodiert, erhöht ist oder die Expressionsregulationssequenz so modifiziert ist, dass die intrazelluläre Expression des Gens oder Operons verstärkt sein sollte, und welches die Fähigkeit zur Produktion von L-Threonin aufweist.

3. Bakterium der Gattung Escherichia nach Anspruch 2, wobei das Threoninoperon aus thrABC besteht.

4. Bakterium der Gattung Escherichia nach Anspruch 1, worin die Aktivität eines Enzyms oder von Enzymen, kodiert durch das ilv-Operon oder einem Teil davon, **dadurch** verstärkt ist, dass die Kopienzahl eines Gens oder Operons, das für das jeweilige Enzym kodiert, erhöht ist oder eine Expressionsregulationssequenz so modifiziert ist, dass die intrazelluläre Expression des Gens oder Operons verstärkt sein sollte, und welches die Fähigkeit zur Produktion von L-Isoleucin aufweist.

5. Bakterium der Gattung Escherichia nach einem der Ansprüche 1 bis 4, wobei die Aspartaseaktivität **dadurch** verstärkt ist, dass die Kopienzahl eines Gens oder Operons, das für das jeweilige Enzym kodiert, erhöht ist oder die Expressionsregulationssequenz so modifiziert ist, dass die intrazelluläre Expression des Gens oder Operons verstärkt sein sollte.

6. Bakterium der Gattung Escherichia nach einem der Ansprüche 1 bis 5, wobei jedes der Gene oder Operons von einem Bakterium der Gattung Escherichia abgeleitet ist.

7. Verfahren zur Produktion von L-Threonin oder L-Isoleucin, welches das Kultivieren eines Bakteriums der Gattung Escherichia nach einem der Ansprüche 1 bis 6 in einem Medium umfasst.

## Revendications

1. Bactérie appartenant au genre *Escherichia,* dotée de la capacité de produire la L-thréonine ou la L-isoleucine, et dans laquelle l'activité de la phosphoénolpyruvate carboxylase et l'activité de la transhydrogénase intracellulaires sont accrues en augmentant le nombre de copies d'un gène ou opéron codant pour chaque enzyme, ou en modifiant une séquence de régulation de l'expression de sorte à ce que l'expression intracellulaire du gène ou opéron soit accrue.

2. Bactérie appartenant au genre *Escherichia* selon la revendication 1, dans laquelle l'activité d'une enzyme ou d'enzymes codées par l'opéron thréonine ou une partie de celui-ci est accrue, en augmentant le nombre de copies d'un gène ou opéron codant pour chaque enzyme, ou en modifiant une séquence de régulation de l'expression de sorte à ce que l'expression intracellulaire du gène ou opéron soit accrue, et qui est dotée d'activité productrice de L-thréonine.

3. Bactérie appartenant au genre *Escherichia* selon la revendication 2, dans laquelle l'opéron thréonine consiste en *thrABC.*

4. Bactérie appartenant au genre *Escherichia* selon la revendication 1, dans laquelle l'activité d'une enzyme ou d'enzymes codées par l'opéron *ilv* ou une partie de celui-ci est accrue en augmentant le nombre de copies d'un gène ou opéron codant pour chaque enzyme, ou en modifiant une séquence de régulation de l'expression de sorte à ce que l'expression intracellulaire du gène ou opéron soit accrue, et qui est dotée d'activité productrice de L-isoleucine.

5. Bactérie appartenant au genre *Escherichia* selon l'une quelconque des revendication 1-4, dans laquelle l'activité de l'aspartase est accrue en augmentant le nombre de copies d'un gène ou opéron codant pour chaque enzyme, ou en modifiant une séquence de régulation de l'expression de façon que l'expression intracellulaire du gène ou opéron soit accrue.

6. Bactérie appartenant au genre *Escherichia* selon l'une quelconque des revendication 1-5, dans laquelle chacun d'entre le gène ou l'opéron provient d'une bactérie appartenant au genre *Escherichia.*

7. Procédé de production de L-thréonine ou de L-isoleucine, qui comprend la culture d'une bactérie appartenant au genre *Escherichia* selon l'une quelconque des revendications 1-6 dans un milieu.


### Sequence Listing

<110> Ajinomoto Co., Inc.
<120> Method for Producing Threonine and Isoleucine
<130> EPA-53708
<150> JP 2000-244921
   <151> 2000-08-11
<160> 6
<170> Patent In Ver. 2.0
<210> 1
   <211> 20
   <212> DNA
   <213> Artificial Sequence
<220>
   <223> Description of Artificial Sequence:primer
<400> 1
   tgatcagcga aacactttta 20
<210> 2
   <211> 19
   <212> DNA
   <213> Artificial Sequence
<220>
   <223> Description of Artificial Sequence:primer
<400> 2
   cagcaaacta tgatgagaa 19
<210> 3
   <211> 22
   <212> DNA
   <213> Artificial Sequence
<220>
   <223> Description of Artificial Sequence:primer
<400> 3
   taacatcact gagatcatgt tg 22
<210> 4
   <211> 21
   <212> DNA
   <213> Artificial Sequence
<220>
   <223> Description of Artificial Sequence:primer
<400> 4
   tcttttcttg catcttgttc g 21
<210> 5
   <211> 22
   <212> DNA
   <213> Artificial Sequence
<220>
   <223> Description of Artificial Sequence:primer
<400> 5
   tctgtttctc aagattcagg ac 22
<210> 6
   <211> 19
   <212> DNA
   <213> Artificial Sequence
<220>
   <223> Description of Artificial Sequence:primer
<400> 6
   cgccggtaaa ccaaaaccc 19

### Sequence Listing

<110> Ajinomoto Co., Inc.
<120> Method for Producing Threonine and Isoleucine
<130> EPA-53708
<150> JP 2000-244921
   <151> 2000-08-11
<160> 6
<170> PatentIn Ver. 2.0
<210> 1
   <211> 20
   <212> DNA
   <213> Artificial Sequence
<220>
   <223> Description of Artificial Sequence:primer
<400> 1
   tgatcagcga aacactttta 20
<210> 2
   <211> 19
   <212> DNA
   <213> Artificial Sequence
<220>
   <223> Description of Artificial Sequence:primer
<400> 2
   cagcaaacta tgatgagaa 19
<210> 3
   <211> 22
   <212> DNA
   <213> Artificial Sequence
<220>
   <223> Description of Artificial Sequence:primer
<400> 3
   taacatcact gagatcatgt tg 22
<210> 4
   <211> 21
   <212> DNA
   <213> Artificial Sequence
<220>
   <223> Description of Artificial Sequence:primer
<400> 4
   tcttttcttg catcttgttc g 21
<210> 5
   <211> 22
   <212> DNA
   <213> Artificial Sequence
<220>
   <223> Description of Artificial Sequence:primer
<400> 5
   tctgtttctc aagattcagg ac 22
<210> 6
   <211> 19
   <212> DNA
   <213> Artificial Sequence
<220>
   <223> Description of Artificial Sequence:primer
<400> 6
   cgccggtaaa ccaaaaccc 19